# NEUE EUROPÄISCHE PATENTSCHRIFT

(11) **EP 0 722 750 B2**
(45) Veröffentlichungstag und Bekanntmachung des Hinweises auf die Entscheidung über den Einspruch: **28.03.2007**
(45) Hinweis auf die Patenterteilung: 28.07.1999
(21) Anmeldenummer: 96100553.5
(22) Anmeldetag: 16.01.1996
(51) Int. Cl.: A61N 5/06, A61H 39/00

(54) **Softlaser mit integriertem Punktfinder für Akupunkturpunkte**
Softlaser with integrated acupuncture prints localizer
Laser atténué, comprenant un repérage intégré des prints d'acupuncture

(30) Priorität: 17.01.1995 AT 5995
(43) Veröffentlichungstag der Anmeldung: 24.07.1996
(73) Patentinhaber: Myles Limited, Douglas (Isle of Man) IM1 4LS (GB)
(72) Erfinder: Nidetzky, Leopold J., Dr., A-1210 Wien (AT)
(74) Vertreter: Schulz, Rütger

(56) Entgegenhaltungen:
- EP-A- 0 079 615
- EP-A- 0 416 150
- EP-A- 0 437 636
- EP-A- 0 465 459
- EP-A- 0 495 757
- WO-A-95/03089
- DE-A- 3 226 507
- J.L. DIAZ MORERA,A. COMBARRO : "Lasermed-1 and lasermed-101 equipments for therapeutic treatments with low power He-Ne lasers" TRENDS IN QUANTUM ELECTRONICS, Bd. 34, Nr. 7-9, 29.August 1988 - 4.September 1988, BUCHAREST(ROMANIA), Seiten 1087-1089, XP002043432

## Beschreibung

Die Erfindung betrifft eine Vorrichtung zum Aufsuchen, von Akupunkturpunkten mittels Hautwiderscandsmessung und zur anschließenden Akupunktur mittels Laserlicht sowie zur Biostimulation, mit
einem handgriffelartigen Gehäuse,
einer darin aufgenommenen Laserdiodeneinheit des Laserschutzklasse 3a mit zugeordneter Schalteinrichtung,
einer vor der Strahlmündung der Laserdiodeneinheit am Gehäuseende angeordneten Tastelektrode,
einer Gegenelektrode auf dem Gehäuse, und
einer an die Tastelektrode und die Gegenelektrode angeschlossenen Meß- und Anzeigeeinrichtung mit einstellbarer Empfindlichkeit für den zwischen Tast- und Gegenelektrode gemessenen Hautwiderstand.

Eine Vorrichtung dieser Art, allerdings mit einer Lumineszenzdiode anstelle einer Laserdiode, ist aus der US 4 535 784 bekannt. Bei dieser Vorrichtung besteht die Tastelektrode aus einem direkt auf die Diode aufgebrachten Ring (Fig. 2, 3), einer direkt auf die Diode aufgebrachten, diese verkapselnden, zentral durchbrochenen Hülse (Fig. 4) bzw. einem in die Diode direkt eingegossenen, an der Vorderseite der Laserdiode vorragenden Kontaktdraht (Fig. 5). Es ist ersichtlich, daß in jedem der genannten Fälle beträchtliche Lichtleistungsverluste in Kauf genommen werden müssen, weil nur ein Bruchteil der emittierten Lichtenergie durch die vom Punktsucher erfaßte Fläche geht. Bei der Variante der Fig. 5 geht die Lichtenergie sogar gerade nicht durch die vom Punktsucher geortete Fläche.

Aus der EP-A-0 437 636 ist eine andere Gattung von kombinierten Akupunkturpunktsuche- und Bestrahlungsvorrichtung bekannt, mit einem handgriffelartigen Gehäuse, in dem eine reflektorgefaßte Halogenlampe zur Bestrahlung enthalten ist, deren Licht durch die Axialöffnung einer etwa konusförmigen, zur Punktsuche bestimmten Tastelektrode ausgesandt wird. Zur Sammlung des von der Halogenlampe ausgesandten Lichtes an der Tastelektrodenöffnung ist die Tastelektrode selbst als sogenanntes "Focon" ausgebildet, das durch Innenreflexion an den konischen Innenwänden im Zusammenwirken mit dem Reflektor der Halogenlampe das Licht zur Ausgangsöffnung als "einzigem Ausgang" der Gegenspiegelanordnung lenken soll. Das Focon bzw. die Tastelektrode ist entweder vollständig mit Glas gefüllt, so daß offensichtlich die Grenzwinkel-Totalreflexion zur Innenreflexion herangezogen werden soll, oder gänzlich hohl und aus reflektierendem Metall gefertigt. Es ist ersichtlich, daß diese Art der Lichtsammlung Absorptionsverlusten an den Innenwänden des Focons unterliegt, was lediglich zu einer Erwärmung derselben führt.

Aus der EP-A-0 495 757 ist wiederum eine Vorrichtung bekannt, bei welcher zwar ein Laserstrahl von einer Laserdiode erzeugt und letztlich durch eine ringförmige Tastelektrode hindurchgeführt wird, jedoch dazwischen Ober einen Lichtleiter geleitet wird, der beim Ein- und Auskuppelvorgang nicht unbeträchtliche Laserenergie absorbiert, wobei der Laserstrahl überdies nicht fokussiert am Behandlungsort auftrifft.

Aus der EP-A-0 416 150 ist ein Laserakupunkturgerät mit einer Laserdiode bekannt, deren Lichtstrahl über eine Fokussierlinse an einer Stirnseite des händgriffelartigen Gerätes durch eine Öffnung in einem Sensorring fokussiert austritt. Der Sensorring ist großflächig und dient als Einschaltsperre, um festzustellen, ob das Gerät an der Haut des Patienten anliegt, was die Freischaltung des Laserstrahles ermöglicht. Zur Akupunkturpunktsuche ist die Vorrichtung ungeeignet.

Die Erfindung setzt sich zum Ziel, eine verbesserte kombinierte Akupunfiturpunldsüche- und Laserakupunktur-Vorrichtung zu schaffen. Dieses Ziel wird bei einer Vorrichtung der einleitend genannten Art erfindungsgemäß dadurch erreicht.
daß kein Lichtleiter verwendet wird und
daß die Tastelektrode im wesentlichen konusförmig ausgebildet ist und eine axiale Bohrung für den Durchtritt des Laserstrahles bis zur Austrittsöffnung an der Spitze der Tastelektrode aufweist,
wobei zwischen Laserdiodeneinheit und Tastelektrode eine Fokussierlinse angeordnet ist, die einen Brennpunkt in der Ebene der Austrittsöffnung erzeugt, und
wobei die Bohrung der Tastelektrode sich in Richtung zur Austrittsöffnung hin verjüngt.

Beim vorliegenden Gerät ist somit die Funktion der Akupunkturpunkte Auffindung mit jener der Akupunkturbehandlung selbst, und zwar mit Hilfe eines Laserstrahles, kombiniert, wobei zwischen diesen beiden Vorgängen das Gerät nicht vom jeweiligen Akupunkturpunkt abgehoben werden muß. Dadurch kann die Akupunkturbehandlung genau an jener Stelle erfolgen, die zuvor als Akupunkturpunkt exakt lokalisiert worden ist, wobei auch von Bedeutung ist, daß der Laserstrahl direkt durch eine Öffnung in der Suchelektrode austritt und der Fokus des Laserstrahls in der Ebene dieser Suchelektrode, die noch in Kontakt mit der Haut des Benutzers steht, gelegen ist

Die vollständige Ausnützung aller zur Verfügung stehenden Laserenergie ist besonders wichtig bei der Verwendung von Laserdioden maximal der Lasershutzklasse 3a, die also nach Anleitung durch den Arzt dem Patienten ausgehändigt und von diesem selbst in Betrieb genommen werden können. In dieser Laserschutzktasse ist die Ausgangsleistung der Laserdiode auf 3 mW begrenzt, so daß selbst kleine Leitungsabstriche die Stimulationswirkung auf den Akupunkturpunkt beeinträchtigen würden.

Das Gerät eignet sich besonders vorteilhaft zur schmerzfreien Selbsthilfe bei Schmerzen bzw. Krankheiten, bei denen eine Akupunkturbehandlung wirksam ist. Das Gerät kann auch zur Biostimulation, z.B. für die Behandlung von Herpes simplex oder von einzelnen Akne-Punkten, erfolgreich eingesetzt werden.

Aus fertigungstechnischen Gründen und aus Sicherheitsgründen ist es von Vorteil, wenn die Lasereinheit durch einen an sich bekannten Diodenlaser, vorzugsweise mit einer Wellenlänge von 635 bis 670 nm, gebildet ist. Ein derartiger Diodenlaser ist als handliche Baueinheit erhältlich. Bei der angegebenen Wellenlänge von 635 nm ist eine optimale Wirkung der Akupunktur sichergestellt, da Untersuchungen ergeben haben, daß bei dieser Wellenlänge die Eindringtiefe in Haut bzw. Körpergewebe besonders groß ist. Die Ausgangsleistung des Diodenlasers kann dabei mit Vorteil auf 3 mW begrenzt werden, so daß der Laser in die Laserschutzklasse 3a fällt, so daß keine zusätzlichen Schutzmaßnahmen notwendig sind.

Eine weitere Sicherheitsfunktion ist darin zu sehen, daß der Laserstrahl unmittelbar nach Austritt aus dem Gerät stark divergiert, wozu vorzugsweise die Lasereinheit mit einer Optik kurzer Brennweite ausgerüstet ist. Bei einer derartigen Anordnung ist der Laserstrahl bereits in sehr kurzer Entfernung von der Austrittsöffnung derart weit aufgefächert, daß die Leistung pro Flächeneinheit ausreichend niedrig ist, so daß beispielsweise auch eine Gefährdung der Augen, sollte der Laserstrahl direkt in die Augen gerichtet werden, vermieden werden kann.

Für die Laserakupunktur ist normalerweise ein relativ kurzzeitiges Einwirken der Lasereinheit erforderlich. Für andere Anwendungen, z.B. zur Stimulation bzw. Therapie von organischem Gewebe ("Laserdusche"), ist hingegen ein länger andauernder Betrieb der Lasereinheit, z.B. über mehrere Minuten, gewünscht. Um diese beiden Funktionen zu ermöglichen, ist es daher von Vorteil, wenn die Schalteinrichtung wahlweise einen Tastbetrieb oder einen Dauerbetrieb ermöglicht.

Eine besonders vorteilhafte Ausführungsform der Erfindung zeichnet sich dadurch aus, daß die Tastelektrode im Gehäuse axial gegen die Kraft einer Feder eindrückbar gelagert ist. Die federnd gelagerte Tastelektrode erbringt den Vorteil, daß über die - relativ gering bemessene - Federkraft eine Vereinheitlichung in der Anpreßkraft der Tastelektrode an der jeweiligen Hautstelle erzielt wird, wodurch in der Folge eine Vereinheitlichung für die Hautwiderstandsmessung erreicht werden kann.

Besonders vorteilhaft ist es dabei, wenn erfindungsgemäß die Tastelektrode in der eingedrückten Stellung die Schalteinrichtung für eine Anspeisung der Laserdiodeneinheit betätigt. Dadurch kann nach dem Lokalisieren eines Akupunkturpunktes auf der Haut des Benutzers (wozu bereits ein federndes Einwärtsschieben der Tastelektrode erfolgt) durch stärkeres Eindrükken der Tastelektrode die Lasereinheit zwecks Abgabe des Laserstrahls aktiviert werden. Um gleichzeitig die Hautwiderstands-Meßeinrichtung abzuschalten, ist der Schalter vorteilhafterweise als Umschalter ausgebildet. Beim Abheben des Gerätes von der Hauptpartie und somit beim Zurückbewegen der Tastelektrode zufolge der Federkraft wird der Schalter wieder zurückgestellt, so daß die Lasereinheit abgeschaltet wird.

Vorteilhafterweise ist aus Sicherheitsgründen elektrisch in Reihe zu dem von der Tastelektrode betätigbaren Schalter ein zusätzlicher Betriebsschalter für die Lasereinheit vorgesehen, so daß zum Einschalten der Lasereinheit sowohl ein stärkeres Eindrücken der Tastelektrode als auch ein Betätigen des Betriebsschalters erforderlich ist. In diesem Zusammenhang ist es günstig, wenn der Betriebsschalter für einen Tastbetrieb ausgelegt ist, d.h. als Tast-Druckschafter vorgesehen ist, wodurch nur dann, wenn der Betriebsschalter entgegen einer Federkraft gedrückt gehalten wird, die Lasereinheit aktiviert wird. Sobald der Finger oder Daumen vom Betriebsschalter genommen wird, geht dieser in seinen Aus-Zustand zurück, wodurch die Lasereinheit stromlos wird. Dies hat den Vorteil, daß bei abgelegtem Gerät ein ungewollter Betrieb der Lasereinheit verhindert wird. Um den vorstehend angesprochenen Dauerbetrieb der Lasereinheit zu ermöglichen, kann es zweckmäßig sein, nicht einen gesonderten Dauerbetrieb-Schalter vorzusehen, sondern die Tastelektrode selbst zum dauernden Einschalten der Lasereinheit zu verwenden. Demgemäß ist es besonders günstig, wenn die Tastelektrode in der eingedrückten Stellung mit Hilfe einer Rasteinrichtung arretierbar ist.

Um die Tastelektrode in ihrer ganz zurückgeschobenen Position einfach arretieren zu können, ist es vorteilhaft, wenn die Arretierung durch eine feststellbare Federraste gebildet ist, deren Raststift in lösbaren Eingriff mit der Tastelektrode bringbar ist. Derartige feststellbare Federrasten sind an sich für die verschiedensten Anwendungen bekannt und als Baueinheiten erhältlich, so daß sich eine nähere Erläuterung erübrigt

Von besonderem Vorteil ist es dabei, wenn die Tastelektrode eine Schtitzausnehmung für den Eingriff des Raststiftes aufweist und in diese Schlitzausnehmung ein gehäusefester Führungsstift zur Führung der Tastelektrode bei deren Hin- und Herbewegung zwischen der Ruhestellung und der Betriebsstellung ragt.

Es hat sich auch als vorteilhaft erwiesen, wenn dem Schalter ein mit der Tastelektrode in Eingriff stehender, federnd beweglicher, z.B. lamellenförmiger Schalthebel zu seiner Betätigung zugeordnet ist. Dabei kann der lamellenförmige, federnde Schalthebel beispielsweise in einen Längsschnitt der Tastelektrode eingreifen, so daß er zugleich zur Längsführung derselben dient. Bei vollständigem Zurückschieben der Tastelektrode kommt der Schalthebel am Ende des Längsschlitzes zum Anschlag und beim weiteren Einschieben der Tastelektrode wird er dann von dieser zur Betätigung des Schalters entgegen seiner Federkraft zurückgedrückt.

Im Hinblick auf die Hautwiderstands-Meßfunktion ist es günstig, wenn die Tastelektrode aus Messing besteht und das die Gegenelektrode bildende Gehäuse ein Aluminium-Strangpreßprofilteil ist. Dies stellt auch eine preiswerte Herstellung sicher.

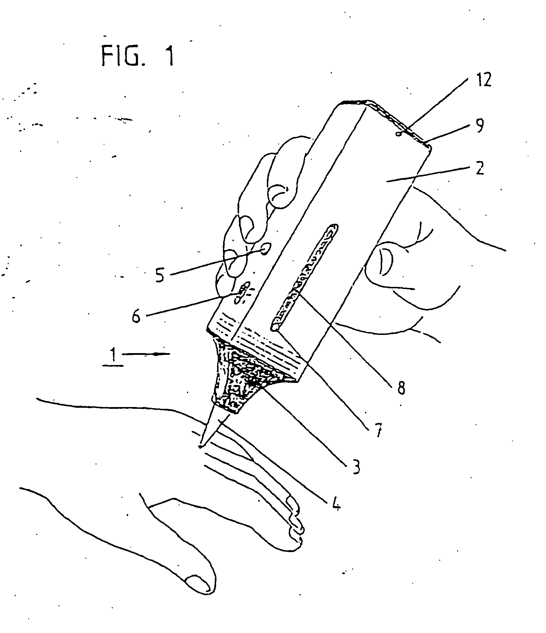

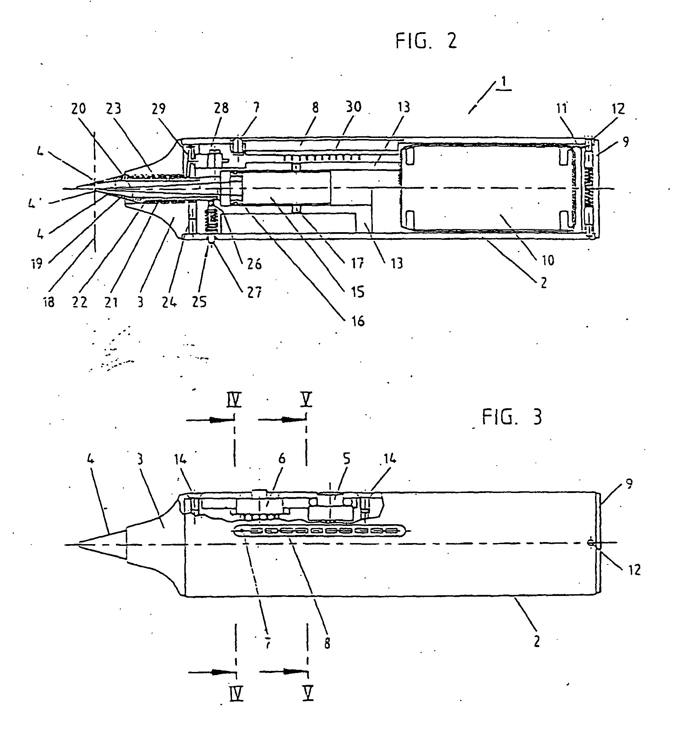

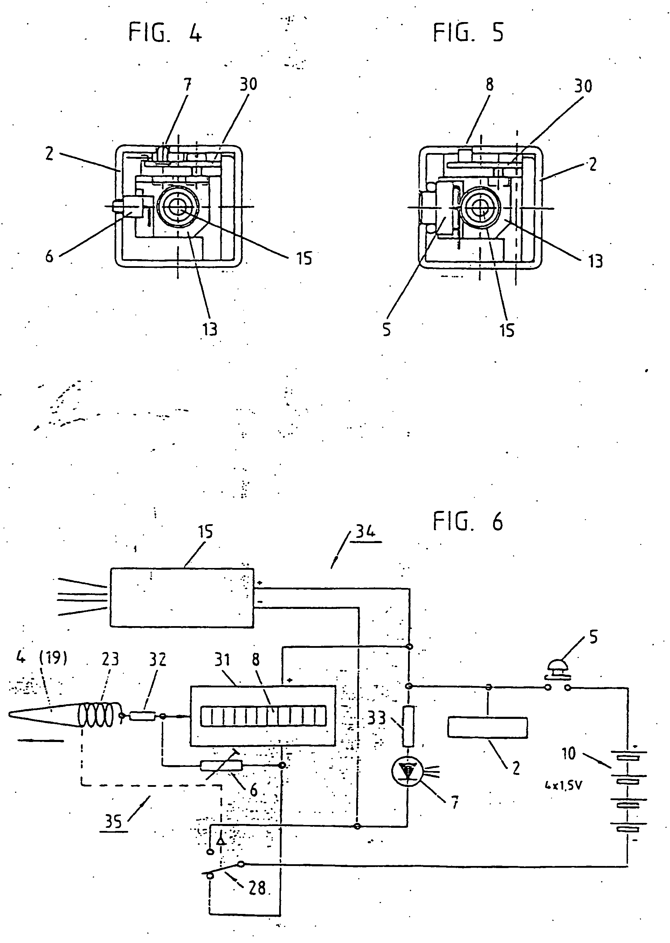

Die Erfindung wird nachstehend anhand eines in der Zeichnung veranschaulichten Ausführungsbeispieles noch weiter erläutert. Im einzelnen zeigen in der Zeichnung: Fig. 1 ein kombiniertes Akupunkturpunkt-Auffindungs- und Laser-Akupunktur-Gerät in einer schaubildlichen Darstellung bei der Anwendung; Fig. 2 einen Längsschnitt durch dieses Gerät, wobei die durch einen Hohlteil gebildete Tast- bzw. Suchelektrode in der oberen Hälfte der Darstellung in der ausgeschobenen Ruhestellung in der unteren Zeichnungshälfte in der eingeschobenen Betriebsstellung veranschaulicht ist; Fig. 3 eine teilweise aufgebrochene Ansicht des Gerätes in einer um 90° gegenüber der Darstellung in Fig. 2 verdrehten Lage; Fig. 4 eine schematische Schnittdarstellung im wesentlichen gemäß der Linie IV-IV in Fig. 3; Fig. 5 eine weitere schematische Schnittdarstellung im wesentlichen gemäß der Linie V-V in Fig. 3; und Fig. 6 ein elektrisches Schaltbild des Gerätes.

In Fig. 1 ist ein allgemein mit 1 bezeichnetes Gerät zum Aufsuchen von Akupunkturpunkten ebenso wie zur Laser-Akupunktur beim Gebrauch veranschaulicht, wobei das Gerät 1 allgemein in der Art eines in sich geschlossenen Griffel-Handgeräts, mit einem länglichen, beispielsweise im Querschnitt quadratischen Gehäuse 2 ausgebildet ist, welches z.B. aus einem Aluminium-Strangpreßprofil, das zumindest bereichsweise aluminisiert oder mit einer anderen leitfähigen Überzugsschicht versehen ist, und welches sich an seinem vorderen Ende in einem sich verjüngenden, ungefähr prismatischen Kunststoff-Lagerteil 3 fortsetzt, der zur längsverschieblichen Lagerung einer gefederten, elektrisch leitenden Tast-Meßspitze 4 dient, die hinsichtlich ihrer Ausbildung und Funktion nachstehend anhand der Fig. 2 bis 5 noch näher erläutert werden soll.

Das Gerät 1 ist mit einem durch das Gehäuse 2 nach außen ragenden Betriebsschalter 5 sowie mit einem Empfindlichkeits-Schiebeschafter 6, z.B. mit drei Stellungen entsprechend drei Empfindlichkeitsbereichen für unterschiedliche Hautwiderstände, ausgestattet, und weiters sind zwecks Anzeige ein Laser-Kontrollampchen 7 sowie eine durch eine Reihe von Leuchtdioden gebildete Meß-Anzeigeeinrichtung 9 vorgesehen; am hinteren Ende ist das Gehäuse 2 des Gerätes 1 mit einem Deckel 9 abgeschlossen, wobei dieser Deckel 9 beispielsweise im Gehäuse 2 eingeschnappt wird und ein Batteriefach mit einem Batterieblock 10 (siehe Fig 2) abschließt. Gemäß Fig 2 ist der Deckel 9 mit einem federbelasteten Schnapp- oder Raststift 11 ausgerüstet, der in eine entsprechende Gehäusebohrung 12 einschnappt, wenn der Deckel 9 auf das Gehäuseende aufgeschnappt wird.

Der das Gehäuse 2 an der Stirnseite verlängernde Lagerteil 3 ist beispielsweise Teil eines Kunststoff-Montagekörpers 13, der im Inneren des Gehäuses 2 verschiedene elektrische und mechanische Komponenten des Geräts 1 trägt. Dieser Montagekörper 13 kann beispielsweise von der Stirnseite her in das Gehäuse 2 eingeschoben und mit Hilfe von Schrauben 14 (siehe Fig. 3) darin fixiert werden. Es sei erwähnt, daß der Montagekörper 13 aus Fig. 2, 4 und 5 nur schematisch und teilweise ersichtlich ist, jedoch ist aus den Darstellungen in Fig. 2 bis 5 die gegenseitige Zuordnung und Anordnung der einzelnen Komponenten, die nachstehend näher zu erläutern sind, klar ersichtlich, wodurch sich auch die Ausbildung des Montagekörpers 13 im einzelnen für den Fachmann ergibt.

Im Inneren des Montagekörpers 13 ist eine Laserdioden- bzw. Dioden-Lasereinheit 15 mit einer Linse 16 angeordnet, wobei in Bohrungen des Montagekörpers 13 eingesetzte Justierschrauben 17 eine genaue axiale Einstellung des Diodenlasers 15 ermöglichen. Im Betrieb, bei eingedrückter Tast-Meßspitze 4 (siehe die Darstellung in der unteren Hälfte von Fig. 2), gibt die Dioden-Lasereinheit 15 einen durch die Linse 16 gebündelten Laserstrahl ab, der durch das Innere des Suchelektroden-Hohlteils 19 hindurch und an der Stirnseite der Meßspitze 4 durch eine Austrittsöffnung 4' austritt, und dessen Fokus in der Ebene 18 der Stirnseite der Meßspitze 4, d.h. der Suchelektrode liegt. Der Laserstrahl ist dabei in Fig. 2 bei 20 angedeutet.

Bei dieser Suchelektrode handelt es sich um einen Hohlteil 19, dessen vorderer Abschnitt konusförmig ist und die genannte Meßspitze 4 bildet, die aus dem Gehäuse 2 bzw. Lagerteil 3 vorsteht und in Stirnansicht allgemein punkt- bzw. - wegen der Austrittsöffnung 4' - allgemein ringförmig, mit kleinem Durchmesser (in der Größenordnung von 1 mm oder weniger), ist, wobei an diese Konus-Meßspitze 4 am hinteren Ende ein rohrförmiger Lagerabschnitt 21 über eine Schulter 22 anschließt. An dieser Schulter oder diesem Absatz 22 liegt eine Schraubenfeder 23 an, die sich mit ihrem anderen Ende an einem aus der Zeichnung nicht näher angegebenen radial einwärts abstehenden flanschartigen Vorsprung des Lagerteils 3 abstützt, wodurch der Hohlteil 19 radial einwärts entgegen der Federkraft der Schraubenfeder 23 verschoben werden kann, wie dies schematisch in Fig. 2 durch die verschiedenen Stellungen des Hohlteils 19 in der oberen bzw. unteren Zeichnungshälfte erkennbar gemacht wurde.

Der rohr- oder hülsenförmige Lagerabschnitt 21 weist in seinem hinteren Endbereich zwei einander diametral gegenüberliegende, in der Zeichnung nicht näher angegebene Längsschlitze auf, wobei in den einen Längsschlitz ein Führungs- und Anschlagstift 24 ragt, der einerseits eine Verdrehsicherung für den Hohlteil 19 bildet und die gefederte Längsbewegung desselben führt, und der andererseits als Anschlag für die Begrenzung der Einwärtsbewegung des Hohlteils 19 entgegen der Kraft der Feder 23 dient. Der Längsschlitz, in den dieser Führungsstift 24 eingreift, ist derart angeordnet, daß bei voll einwärts geschobenem Hohlteil 19, siehe Fig. 2, untere Hälfte, weiters eine Arretierung 25 mit einem Raststift 26 in den längsschlitz, an dessen hinterem, Weiter innen liegenden Ende, einrasten kann, wenn die Arretierung 25 über einen durch das Gehäuse 2 nach außen ragenden Betätigungszapfen 27 aktiviert wird.

Über diesen Zapfen oder Stift 27 kann die Arretierung 25 auch wieder gelöst werden, um so den Suchelektroden-Hohlteil 19 wieder für eine Auswärtsbewegung zufolge der Federkraft (Feder 23) freizugeben.

Der Arretierung 25 diametral gegenüber liegt ein Schalter 28 für die Laserfunktion. Dieser Schalter 28 wird mit Hilfe eines federnden, lamellenartigen Schalthebels 29 betätigt, der in den anderen, gegenüberliegenden Längsschlitz des Suchelektroden-Hohlteils 19, d.h. dessen Lagerabschnitt 21, eingreift, und der beim Einwärtsschieben des Hohlteils 19 durch das vordere Schlitzende, an dem er zur Anlage kommt, nach hinten verschwenkt wird, um so den Schalter 28 zu bestätigen.

Über nicht näher ersichtliche Distanzstifte, Schrauben oder dergl. trägt der Montagekörper 13 weiters eine Leiterplatte 30 mit der Meßschaltung (31; Fig. 6) für die Hautwiderstandsmessung, mit den Schaltkreisen für die Kontrollampe 7 und die Anzeigeenrichtung 8 für die Hautwiderstandsmessung und mit den erforderlichen Schaltungsteilen für den Diodenlaser 15. Die elektrischen Verbindungen sind dabei in Fig. 2 der Übersichtlichkeit halber nicht gezeigt, ergeben sich jedoch aus der Darstellung von Fig 6.

In Fig. 6 ist in einem ganz schematischen Blockschaltbild die elektrische Schaltung des vorliegenden Gerätes 1 veranschaulicht, wobei die auf der Leiterplatte 30 (siehe Fig. 2) vorgesehene Meßschaltung 31 nur ganz allgemein mit einem Block veranschaulicht ist. Diese Hautwiderstands-Meßschaftung 31 ist mit ihrem Eingang über einen Widerstand 32 mit der Suchelektrode, d.h. mit der Konus-Meßspitze 4, elektrisch verbunden, und sie steuert die LEDZeilen-Anzeigeeinrichtung 9 entsprechend dem gemessenen Hautwiderstand an, wobei je nach gemessenem Hautwiderstand mehr oder weniger Dioden dieser LED-Zeile zum Leuchten gebracht werden. Die Empfindlichkeit der Meßschaltung 31 kann wie erwähnt über den Empfindtichkeitsschatter 6 eingestellt werden, (bei dem es sich vorzugsweise um einen Einstellwiderstand, z.B. ein Einstellpotentiometer mit drei Stellungen, handelt.

Die Gegenelektrode für die Hautwiderstandmessung wird durch das elektrisch leitende Gehäuse 2 gebildet, welches im Schaltbild von Fig. 6 nur ganz schematisch mit einem Block veranschaulicht ist. Weiters sind aus Fig. 6 der Batterieblock 10, der beispielsweise aus vier 1,5 Volt-Zellen aufgebaut ist (es können auch wiederaufladbare Akku-Zellen verwendet werden), sowie der durch einen Tast-Schalter gebildete Betriebsschalter 5 ersichtlich. Der Schalter 28 ist, wie aus Fig. 6 erkennbar ist, als Umschalter ausgebildet. Der Schalter 28 befindet sich normalerweise in der in Fig. 6 gezeigten Stellung, in der die Meßschaltung 31 an den Batterieblock 10 angeschlossen ist (sofern der Betriebsschalter 5 gedrückt wird), um so die Hautwiderstandsmessung vorzunehmen. Wie erwähnt wird dieser Schalter 28 mechanisch durch die Suchelektrode, d.h. die Meßspitze 4, bei deren Zurückschieben über den Schalthebel 29 (siehe Fig 2) betätigt, wobei dann die Meßschaltung 31 vom Batterieblock 10 getrennt und die Lasereinheit 15 an diesen Batterieblock 10 angeschlossen wird. Dabei wird parallel zur Lasereinheit 15 die durch eine LED gebildete Kontrollampe 7 für den Laser 15 über einen Vorwiderstand 33 an Spannung gelegt.

Ganz allgemein ist die Laserschaltung mit der Lasereinheit 15 und dem zugehörigen Kontrollampen-Schaltzweig in Fig. 6 mit 34 angegeben, wogegen die HautwiderstandsMeßeinrichtung mit der Suchelektrode bzw. der Meßspitze 4 und der eigentlichen Meßschaltung 31 mit 35 bezeichnet ist. Die Meßschaltung 31 ebenso wie die Lasereinheit 15 können in an sich herkömmlicher Weise, etwa im Prinzip ähnlich wie in den eingangs genannten Schriften geoffenbart, aufgebaut sein, so daß sich eine weitere Erläuterung hievon erübrigen kann. Insbesondere kann die Meßschaltung 31 mit einem nicht näher veranschaulichten Operationsverstärker aufgebaut werden, und als Lasereinheit 15 kann eine im Handel erhältliche Diodenlasereinheit, beispielsweise der Laserklasse 3a, mit einer Beschränkung der Laserleistung auf 3 mW und mit einer Wellenlänge 670 nm, verwendet werden.

Im Betrieb wird z.B. wie allgemein in Fig. 1 veranschaulicht ein Hautbereich mit der - entgegen der Federkraft etwas eingedrückten - Meßspitze 4 des in einer Hand gehaltenen Geräts 1 abgetastet, um den genwünschten Akupunktupunkt durch Hautwiderstandsmessung zu eruieren.

Bei Anzeige eines Maximums an der Anzeigeeinrichtung 8 ist der gewünschte Akupunkturpunkt lokalisiert, und das Gerät 1 wird nicht mehr weiter über den Hautbereich bewegt, sondern an dieser aufgefundenen Stelle fest auf die Haut aufgedrückt, so daß der SuchelektrodenHohfteil 19 bis zum Anschlag einwärts geschoben wird, wodurch der Schalter (Umschalter) 28 bestätigt wird, um die Hautwiderstands-Meßeinrichtung 35 ab- und die Leisereinheit 15 anzuschalten. Dabei ist es selbstverständlich notwendig den Betriebsschalter 5 niedergedrückt zu hatten.

Sofern eine sogenannte "Laserdusche" gewünscht wird, kann in voll einwärts geschobenen Position des Suchelektroden-Hohlteils 19 die Arretierung 25 über den Stift 27 betätigt werden, so daß die Lasereinheit 15 in einen Dauerbetrieb (anstatt des zuvor beschriebenen ("Tastbetriebs") versetzt wird, in dem das Gerät 1 von der zu behandelnden Hautpartie abgehoben werden kann, ohne daß der Laserstrahl 20 unterbrochen wird.

Wenn die Erfindung vorstehend anhand von besonders bevorzugten Ausführungsbeispielen näher erläutert wurde, so sind doch selbstverständlich Abwandlungen und Modifikationen im Rahmen der Erfindung möglich. So ist es beispielsweise denkbar, anstatt des beschriebenen und in den Fig. 4 und 5 veranschaulichten, im Querschnitt quadratischen Gehäuses 2 ein zylindrisches Gehäuse mit kreisförmigem öder elliptischem Querschnitt zu verwenden, wobei dann der Montagekörper 13 sowie die Anordnung der entsprechenden Schalter und Anzeigeelemente anzupassen wäre. Auch ist es an sich denkbar, für den Betriebsschalter 5 anstatt eines Tast-Schalters einen Aus-/Ein-Schalter, mit zwei festen Stellungen, zu verwenden, da der Schalter 28 als Sicherheitsschalter normalerweise die Lasereinheit 15 von der Spannungseinheit, d.h. vom Batterieblock 10, getrennt hält. Im Hinblick auf die Arretierungsfunktion, mit Hilfe der beschriebenen Arretierung 25, wird jedoch die Ausbildung des Betriebsschafters 5 als Tast-Schalter bevorzugt, da bei arretierter Meßspitze 4 ansonsten die Lasereinheit 15 irrtümlich dauernd aktiviert sein könnte.

## Patentansprüche

1. Vorrichtung zum Aufsuchen von Akupunkturpunkten mittels Hautwiderstandsmessung und zur anschließenden Akupunktur mittels Laserlicht sowie zur Biostimulation, mit
einem handgriffelartigem Gehäuse (2),
einer darin aufgenommenen Laserdiodeneinheit (15) der Laserschutzklasse 3a mit zugeordneter Schalteinrichtung (5, 28),
einer vor der Strahlmündung der Laserdiodeneinheit (15) am Gehäuseende angeordneten Tastelektrode (4),
einer Gegenelektrode auf dem Gehäuse, und einer an die Tastelektrode (4) und die Gegenelektrode angeschlossenen Meß- und Anzeigeeinrichtung (31) mit einstellbarer Empfindlichkeit für den zwischen Tast- und Gegenelektrode gemessenen Hautwiderstand,
wobei die Anzeigeeinrichtung (31) als optische Anzeigeeinrichtung (31) ausgestaltet ist,
**dadurch gekennzeichnet,**
**dass** bein Lichtleiter verwendet wird und dass die Tastelektrode (4) im Wesentlichen konusförmig ausgebildet ist und eine axiale Bohrung für den Durchtritt des Laserstrahles (20) bis zur Austrittsöffnung (4') an der Spitze der Tastelektrode (4) aufweist,
wobei zwischen Laserdiodeneinheit (15) und Tastelektrode (4) eine Fokussierlinse (16) angeordnet ist, die einen Brennpunkt in der Ebene (18) der Austrittsöffnung (4') erzeugt,
und
wobei die Bohrung der Tastelektrode (4) sich in Richtung zur Austrittsöffnung (4') hin verjüngt.

2. Vorrichtung nach Anspruch 1, **dadurch gekennzeichnet, dass** die Austrittsöffnung (4') der Bohrung einen Durchmesser in der Größenordnung von 1 mm oder weniger hat.

3. Vorrichtung nach einem der Ansprüche 1 oder 2, **dadurch gekennzeichnet, dass** die Laserdiodeneinheit (15) im Gehäuse (2) mittels Justierschrauben (17) axial auf die Bohrung ausrichtbar gelagert sind.

4. Vorrichtung nach einem der Ansprüche 1 bis 3, **dadurch gekennzeichnet, dass** die Fokussierlinse (16) von kurzer Brennweite ist.

5. Vorrichtung nach einem der Ansprüche 1 bis 4, **dadurch gekennzeichnet, dass** die Schalteinrichtung (5, 28) in der Anspeisestellung der Laserdiodeneinheit (15) die Mess- und Anzeigeeinrichtung (31) abschaltet.

6. Vorrichtung nach einem der Ansprüche 1 bis 5, **dadurch gekennzeichnet, dass** die Tastelektrode (4) im Gehäuse (2) axial gegen die Kraft einer Feder (23) eindrückbar gelagert ist.

7. Vorrichtung nach Anspruch 6, **dadurch gekennzeichnet, dass** die Tastelektrode (4) in der eingedrückten Stellung die Schalteinrichtung (28) für eine Anspeisung der Laserdiodeneinheit (15) betätigt.

8. Vorrichtung nach Anspruch 7, **dadurch gekennzeichnet, dass** die Tastelektrode (4) in der eingedrückten Stellung mit Hilfe einer Rasteinrichtung (25) arretierbar ist.

9. Vorrichtung nach einem der Ansprüche 1 bis 8, **dadurch gekennzeichnet, dass** die Mess- und Anzeigeeinrichtung (31) eine LED-Kette (8) zur Anzeige des Hautwiderstandes aufweist.

10. Vorrichtung nach einem der Ansprüche 1 bis 9, **dadurch gekennzeichnet, dass** die Mess- und Anzeigeeinrichtung (31) mit einem Empfindlichkeits-Schiebeschalter (6) ausgestattet ist.

## Claims

1. Apparatus for finding acupuncture points by means of
measuring skin resistance and for then carrying out acupuncture by means of laser light and for biostimulation, with
a stylus-like housing (2),
a laser diode unit (15) received therein, of laser safety class 3a, with associated switching device (5, 28),
a sensing electrode (4) arranged in front of the beam outlet of the laser diode unit (15) on the end of the housing,
a counter electrode on the housing, and
a measuring and display device (31), connected to the sensing electrode (4) and the counter electrode, with adjustable sensitivity for the skin resistance measured between the sensing electrode and the counter electrode, wherein the display device (31) is an optical display
**characterised in that**
no light guide is used,
the sensing electrode (4) is configured substantially conical and has an axial bore through which the laser beam (20) can pass to the exit aperture (4') at the tip of the sensing electrode (4),
there being disposed between the laser diode unit (15) and the sensing electrode (4) a focusing lens (16) which generates a focal point in the plane (18) of the exit aperture (4') and
the bore of the sensing electrode (4) tapering towards the exit aperture (4').

2. Apparatus according to claim 1, **characterised in that** the exit aperture (4') of the bore has a diameter in the order of 1 mm or less.

3. Apparatus according to one of claims 1 or 2, **characterised in that** the laser diode unit (15) is mounted in the housing (2) by means of adjusting screws (17) so as to be able to be aligned axially with the bore.

4. Apparatus according to one of claims 1 to 3, **characterised in that** the focusing lens (16) is of short focal length.

5. Apparatus according to one of claims 1 to 4, **characterised in that** the switching device (5, 28) in the position for feeding the laser diode unit (15) switches off the measuring and display device (31).

6. Apparatus according to one of claims 1 to 5, **characterised in that** the sensing electrode (4) is mounted in the housing (2) so as to be able to be depressed axially against the force of a spring (23).

7. Apparatus according to claim 6, **characterised in that** the sensing electrode (4) in the depressed position actuates the switching device (28) for feeding the laser diode unit (15).

8. Apparatus according to claim 7, **characterised in that** the sensing electrode (4) may be locked in the depressed position with the aid of a locking device (25).

9. Apparatus according to one of claims 1 to 8, **characterised in that** the measuring and display device (31) has an LED chain (8) to display the skin resistance.

10. Apparatus according to one of claims 1 to 9, **characterised in that** the measuring and display device (31) is equipped with a sensitivity slide switch (6).

## Revendications

1. Dispositif pour rechercher des points d'acupuncture à l'aide d'une mesure de la résistance de la peau et pour effectuer ensuite un traitement d'acupuncture à l'aide d'une lumière laser ainsi que pour réaliser une biostimulation, comportant
un boîtier (2) en forme de manche,
une unité à diode laser (15), logée dans ce boîtier, de la classe de protection laser 3a avec un dispositif de commutation associé (5, 28),
une électrode de détection (4) disposée sur l'extrémité du boîtier, en avant de l'ouverture de l'unité à diode laser (15),
une contre-électrode située sur le boîtier, et
un dispositif de mesure et d'affichage (31) raccordé à l'électrode de détection (4) et à la contre-électrode et possédant une sensibilité réglable, pour mesurer et afficher la résistance de la peau mesurée entre l'électrode de détection et la contre-électrode, lequel dispositif d'affichage est un dispositif d'affichage optique
**caractérisé en ce**
**qu'**un conduit de lumière n'est pas utilisé,
l'électrode de détection (4) est agencée sensiblement avec une forme conique et comporte un perçage axial pour le passage du faisceau laser (20) jusqu'à l'ouverture de sortie (4') au niveau de la pointe de l'électrode de détection (4),
dans lequel entre l'unité à diode laser (15) et l'électrode de détection (4) est disposée une lentille de focalisation (16), qui produit un foyer dans le plan (18) de l'ouverture de sortie (4'), et
dans lequel le perçage de l'électrode de détection (4) se rétrécit en direction de l'ouverture de sortie (4').

2. Dispositif selon la revendication 1, **caractérisé en ce que** l'ouverture de sortie (4') du perçage possède un diamètre de l'ordre de 1 mm ou moins.

3. Dispositif selon l'une des revendications 1 ou 2, **caractérisé en ce que** l'unité à diode laser (15) est montée dans le boîtier (2) de manière à pouvoir être alignée axialement sur le perçage, à l'aide de vis d'ajustement (17).

4. Dispositif selon l'une des revendications 1 à 3, **caractérisé en ce que** la lentille de focalisation (16) possède une courte distance focale.

5. Dispositif selon l'une des revendications 1 à 4, **caractérisé en ce que** le dispositif de commutation (5, 28) débranche le dispositif de mesure et d'affichage (31) lorsque l'unité à diode laser (15) est dans la position d'alimentation.

6. Dispositif selon l'une des revendications 1 à 5, **caractérisé en ce que** l'électrode de détection (4) est montée de manière à pouvoir être repoussée axialement dans le boîtier (2) à l'encontre de la force d'un ressort (23).

7. Dispositif selon la revendication 6, **caractérisé en ce que** dans la position enfoncée l'électrode de détection (4) actionne le dispositif de commutation (28) pour une alimentation de l'unité à diode laser (15).

8. Dispositif selon la revendication 7, **caractérisé en ce que** dans l'état enfoncé, l'électrode de détection (4) peut être bloquée à l'aide d'un dispositif d'encliquetage (25).

9. Dispositif selon l'une des revendications 1 à 8, **caractérisé en ce que** le dispositif de mesure et d'affichage (31) possède une chaîne de diodes LED (8) pour l'affichage de la résistance de la peau.

10. Dispositif selon l'une des revendications 1 à 9, **caractérisé en ce que** le dispositif de mesure et d'affichage (31) est équipé d'un commutateur de sensibilité coulissant (6).
